(19) Europäisches Patentamt
European Patent Office
Office européen des brevets

(11) **EP 4 033 242 B1**

(12) **EUROPEAN PATENT SPECIFICATION**

(45) Date of publication and mention
of the grant of the patent:
**18.06.2025 Bulletin 2025/25**

(51) International Patent Classification (IPC):
**G01N 31/12** (2006.01) **G01N 1/22** (2006.01)
**G01N 1/44** (2006.01) **G01N 15/06** (2024.01)
**G01N 21/31** (2006.01)

(21) Application number: **21187069.6**

(22) Date of filing: **21.07.2021**

(52) Cooperative Patent Classification (CPC):
**G01N 1/2205; G01N 15/0625; G01N 31/12;**
G01N 1/44; G01N 21/31; G01N 33/004;
G01N 2001/2223; G01N 2015/0046; Y02A 50/20

(54) **A DEVICE AND A METHOD FOR COMPLETE CARBONACEOUS AEROSOL ANALYSIS IN REAL TIME**

VORRICHTUNG UND VERFAHREN ZUR VOLLSTÄNDIGEN ANALYSE VON KOHLENSTOFFHALTIGEN AEROSOLEN IN ECHTZEIT

DISPOSITIF ET PROCÉDÉ D'ANALYSE COMPLÈTE D'AÉROSOL CARBONÉ EN TEMPS RÉEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.07.2022 Bulletin 2022/30**

(73) Proprietor: **Aerosol d.o.o.**
**1000 Ljubljana (SI)**

(72) Inventors:
• **Gregoric, Asta**
**1356 Dobrova (SI)**
• **Ivancic, Matic**
**1275 Smartno pri Litiji (SI)**
• **Rigler, Martin**
**1000 Ljubljana (SI)**

(74) Representative: **Patentni Biro AF d.o.o.**
**Kotnikova 32, p.p. 2706**
**1001 Ljubljana (SI)**

(56) References cited:
• **RIGLER MARTIN ET AL: "The new instrument using a TC-BC (total carbon - black carbon) method for the online measurement of carbonaceous aerosols", ATMOSPHERIC MEASUREMENT TECHNIQUES, vol. 13, no. 8, 17 August 2020 (2020-08-17), DE, pages 4333 - 4351, XP055877807, ISSN: 1867-1381, DOI: 10.5194/ amt-13-4333-2020**

• **WANG QINGLU ET AL: "Exploring the variation of black and brown carbon during COVID-19 lockdown in megacity Wuhan and its surrounding cities, China", SCIENCE OF THE TOTAL ENVIRONMENT, ELSEVIER, AMSTERDAM, NL, vol. 791, 148226, 4 June 2021 (2021-06-04), pages 1 - 9, XP086735185, ISSN: 0048-9697, [retrieved on 20210604], DOI: 10.1016/ J.SCITOTENV.2021.148226**

• **DUMKA U C ET AL: "Assessment of PM2.5 chemical compositions in Delhi: primary vs secondary emissions and contribution to light extinction coefficient and visibility degradation", JOURNAL OF ATMOSPHERIC CHEMISTRY, SPRINGER, DORDRECHT, NL, vol. 74, no. 4, 15 November 2016 (2016-11-15), pages 423 - 450, XP036371036, ISSN: 0167-7764, [retrieved on 20161115], DOI: 10.1007/S10874-016-9350-8**

**Description**

Field of the invention

The present invention belongs to the field of methods and devices for analysing materials by determining their chemical or physical properties by the use of thermal and optical means, more precisely to the systems especially adapted for detection and quantification of carbonaceous aerosol. The invention relates to a device and a method for complete carbonaceous aerosol analysis in real time.

Background of the invention and the technical problem

Carbonaceous aerosol (CA) represents extreme diversity and a significant fraction of fine particulate matter (PM$_{2.5}$). CA has drawn increasing attention in the scientific community because it acts as a pollutant with critical local, regional, and global importance. It directly impacts public health, visibility, cloud formation, and planetary radiation balance, but the processes are still not fully understood due to their complex chemical and physical properties. Thus, adequate online and high-time resolution apportionment methods of CA are needed to identify the main pollution sources, and point out CA components with the highest impact on public health and climate change processes. It is an aim of the invention to provide a device that will be arranged to analyse CA and determine proportions of as many CA types as possible.

It is commonly accepted that carbonaceous aerosol (CA, also called carbonaceous matter (CM)) includes organic fraction, i.e., organic aerosol (OA, also called organic matter (OM)), and refractory, strong light-absorbing fraction referred to as elemental carbon (EC, determined using a thermal-optical measurement) or as black carbon (BC, determined using an optical measurement). BC and EC are often used interchangeably; nevertheless, in this patent application BC will be used exclusively when describing the refractory strong-absorbing fraction of CA. EC is used only for EC-to-BC intercomparison of thermal-optical and optical method. The BC is chemically inert and has well-defined chemical structure. It is exclusively emitted from incomplete combustion, thus having only primary origin.

OA is made up of many different complex molecular structures and includes not only particulate organic carbon, but also hydrogen, oxygen, nitrogen, and sulphur. In contrast to BC, OA can be volatile and have high oxidative potential. OA is directly emitted to the atmosphere in particulate form as primary organic matter (POA) by combustion and biogenic processes, or it can have a secondary origin (SOA) from the gas-to-particle conversion of (semi)volatile organic compounds in the atmosphere. The sum of BC and POA is known as primary CA, while SOA equals secondary CA. Two pathways of SOA formation are commonly accepted:

- A major formation pathway of SOA is the photooxidation of volatile organic compounds (VOCs) in the afternoon, especially in summer, where hydroxyl radical (OH-) and ozone (Os) play a key role during daytime atmospheric oxidation (He et al., 2021: doi.org/10.1021/acs.est.0c06838).
- The second pathway of SOA formation, especially evident during winter nights, could be explained as aqueous-phase processes when $NO_3$. radical is recognized as a dominant oxidant (He et al., 2021: doi.org/10.1021/acs.est.0c06838). $NO_3$. radical formed by the reaction of nitrogen dioxide $NO_2$ and Os at the presence of high humidity.

The mass of carbon atoms found in CA and OA is called total carbon (TC) and organic carbon (OC). Measurement of total carbon (TC) is performed with devices, which use thermal treatment of sampled air with particles to calculate total carbon based on generated carbon dioxide. Additionally, OC can be further divided to carbon fraction of POA and SOA (POC and SOC, respectively). Two measurement techniques are used for TC/OC determination; thermo-optical OC/EC method and newly developed TC-BC method. Thermo-optical OC/EC method was developed in 1982 by Huntzinker et al. (Huntzicker, J. J., Johnson, R. L., Shah, J. J., and Cary, R. A.: Analysis of Organic and Elemental Carbon in Ambient Aerosols by a thermal-Optical Method, 79-88, 1982) and updated with several changes in thermal protocols (IMPROVE, NIOSH, EUSAAR2). Recently, the simplified TC-BC method was introduced by (Rigler et al., 2020: doi.org/10.5194/amt-13-4333-2020), where OC is determined by subtraction of separately measured TC and BC.

The relationship between OC and OA is called the ambient organic aerosol to organic carbon ratio (OA/OC) and is an important parameter to investigate OA chemical composition. The ratio can vary widely depending on the sources, monitoring location, season, and meteorology. The lower ambient OA/OC ratios are consistent with fresh aerosol emissions from traffic, while the higher values are usually observed for aged ambient oxygenated OA (Chirico et al., 2010: doi.org/10.5194/acp-10-11545-2010). Similarly, the POA/POC and SOA/SOC ratios for primary and secondary OA were introduced by Docherty et al. (2008: doi.org/10.1021/es8008166) and Zhang et al. (2018: doi.org/10.1016/j.jes.2017.12.018).

Organic aerosol can be further divided into light-absorbing OA, also known as brown carbon (BrC), and non-light absorbing OA (OA$_{non-abs}$), both with possible primary and secondary origin (POA$_{BrC}$, SOA$_{BrC}$, POA$_{non-abs}$, SOA$_{non-abs}$,

respectively). In chemistry, brown carbon (Cbrown/BrC) is a brown smoke released by the combustion of organic matter, which absorbs strongly in the ultraviolet wavelengths and less significantly going into the visible wavelengths (Laskin et al., 2015; Moise et al., 2015). It coexists with black carbon when released in the atmosphere. Contrary to the black carbon, brown carbon is emitted mainly by biomass combustion, but also from smoldering fires or coal combustion, from soil, and volatile organic compounds given off by vegetation.

While BC absorption is relatively well characterized, the knowledge on sources and optical properties of BrC remains limited, inducing large uncertainties in the radiative forcing assessment at the global scale (Saleh, 2020; Saleh et al., 2015). Simulation models suggest, however, that brown carbon contributes approximately one fifth of the total atmospheric absorption by aerosols and it may play an important role in photochemistry and the hydrologic cycle, especially over regions dominated by biomass combustion. Being a possible cause of climate change, brown carbon needs to be considered. Therefore, reliable measurements of brown carbon alone have to be provided in addition to other aerosols, so that presence and amounts of each species is determined.

Current devices and methods are directed towards total carbon and black carbon, and fail to focus on brown carbon and other aerosol species. The present invention is based on these considerations. It is thus the aim of the invention to provide a device and a method for complete analysis of carbonaceous aerosol, which will enable detection and quantification of all important carbonaceous aerosol types (components).

State of the art

US2019277819 discloses a device for measuring, in near-real-time, the level of black carbon, brown carbon, organic carbon, total carbon and $CO_2$ in air. The device also provides for a direct calculation of aerosol angstrom coefficient as well as estimation of emissions rates of black carbon or brown carbon from nearby combustion sources. This device determines brown carbon based on transmittance measurements at 370 nm.

The utility model CN209182229 provides an online detection device for light absorption characteristics of water-insoluble brown carbon in atmosphere. An atmospheric sample inlet is positioned on the front side of a continuous liquefaction sampling device; the conveying device is communicated with the continuous liquefaction sampling device so as to input atmospheric fine particles into the continuous liquefaction sampling device; the atomizer is communicated with the continuous liquefaction sampling device and is close to the atmospheric sample inlet; a methanol solution is contained in the atomizer, atomized methanol is conveyed into the continuous liquefaction sampling device, a methanol injection port is communicated with the continuous liquefaction sampling device and located on the rear side of the continuous liquefaction sampling device, and an output port of the continuous liquefaction sampling device is divided into two channels which are communicated with the organic carbon analyzer and the ultraviolet-visible spectrophotometer respectively. The device differs from the present invention in the design as well as in determination of aerosol species. This device does not allow determination of brown carbon.

Recently, Rigler et al. (2020a, doi.org/10.5194/amt-13-4333-2020) introduced a simplified TC-BC method for real-time and high-time resolution TC/BC determination. The carbonaceous aerosol speciation system (CASS) is used for this method, where the thermal approach of Total Carbon analyzer TCA08 (Rigler et al., 2020a) for TC determination is coupled to the optical method of Aethelometer AE33 (Drinovec et al., 2015) for multiwavelength BC measurement. However, this article is silent about determination of important aerosol species and their measurement and/or calculation from measured data.

Wang et al (2021; doi: 10.1016/j.scitotenv.2021.148226) studied the variation of black carbon (BC) and brown carbon (BrC) during COVID-19 lockdown in Wuhan and its surrounding cities in China. The light absorption coefficients of the carbonaceous aerosols were measured by a multi-wavelength aethalometer (Model AE-31, Magee Scientific, USA) with seven-band ($\lambda$ = 370, 470, 520, 590, 660, 880 and 950 nm) in six cities. The flow rate and temporal resolution were set 5 L min-1 and 1 h, respectively. This disclosure relates only to use of aethalometers, which differs from the present invention.

Dumka et al (2017; doi: 10.1007/s10874-016-9350-8) assessed PM2.5 chemical compositions in Delhi. PM2.5 samples have been collected in Delhi, India and analyzed for carbonaceous and inorganic species. PM10 measurements were made simultaneously such that PM10-2.5 could be estimated by difference. This study analyzes the temporal variation of PM2.5 and carbonaceous particles (CP), focusing on identification of the primary and secondary organic aerosol (POA and SOA, respectively). These are not divided with regards to BrC or non-absorbing POA and SOA ($POA_{BrC}$, SOAsrc, $POA_{non-abs}$, $SOA_{non-abs}$), therefore the present invention is different from this disclosure.

Description of the solution of the technical problem

The invention addresses the problem of complete analysis of all aerosol species, not only the most common types as determined by known devices. The technical problem has been solved as defined in the independent claims, while preferred embodiments of the invention are defined in the dependent claims.

The terminology used throughout the text is as follows:

| | |
|---|---|
| CA | Carbonaceous aerosol (also carbonaceous matter, CM, total or complete CA) |
| OA | Organic aerosol (also organic matter, OM) |
| BC | Black carbon |
| EC | Elemental carbon |
| POA | Primary organic aerosol |
| SOA | Secondary organic aerosol (also secondary CA) |
| Primary CA | Sum of BC and POA |
| BrC | Brown carbon (light-absorbing OA) |
| $POA_{BrC,}$ | Primary BrC |
| $SOA_{BrC}$ | Secondary BrC |
| $OA_{non-abs}$ | Non-light-absorbing OA |
| $POA_{non-abs}$ | Primary non-light-absorbing OA |
| $SOA_{non-abs}$ | Secondary non-light-absorbing OA |
| TC | Total carbon = carbon content in CA |
| OC | Organic carbon, the carbon content in OA |
| POC, SOC | The carbon content of POA and SOA |
| OA/OC | Organic aerosol to organic carbon ratio |
| POA/POC | primary organic aerosol to primary organic carbon ratio, |
| SOA/SOC | secondary organic aerosol to secondary organic carbon ratio |
| $Bc_{fl} BC_{bb}$ | Fossil fuel BC component and biomass burning BC component |
| OC/EC, | OC-to-EC-ratio |
| Cvoc | The carbon content of Volataile organic Compounds (VOC) |

The essence of the device for complete carbonaceous aerosol analysis in real time is that the device is a system combining two different instruments, wherein the first instrument measures total carbon (TC) using flash heating of collected aerosol samples and generation of $CO_2$, while the second instrument performs an optical attenuation analysis at preferably 7 wavelengths from near UV (370 nm) to near IR range (950 nm) in order to characterize optical absorption of aerosols accumulated on a glass-fiber/PTFE filter tape. The device, i.e., the system of said instruments, collects and processes collected data of both instruments, wherein said processing may be performed by any of the instruments or by a separate processing means, computer or computer application. The invention allows determination of previously unknown or undetectable aerosol components, namely $POA_{non-abs}$ and $SOA_{non-abs}$, which are primary and secondary non-light-absorbing OA, respectively.

The following aerosol species can be determined based on the operation of both instruments, wherein newly assessed aerosol species are underlined. Parameters written in bold italic are taken from published scientific literature or can be determined by complementary measurement. Among these:

- *b is a* determined proportionality parameter, region or site specific, depend and also on thermal protocol of OC/EC analysis, wherein its usual range is 0.5 to 1.5;

- $MAC_{BC}$, which is 7.77 $m^2$/g;

- $MAC_{BrC}$ is mass absorption cross-section for brown carbon (BrC). The range of possible $MAC_{BrC}$ values for eBrC estimation known from literature is very wide and can take any value between 0.2 - 7.5 $m^2$/g;

- OA/OC ratio is taken from the literature and is usually between 1.2 and 2.5 (Aiken et al., 2008);

- POA/POC ratio is usually around 1.2 to 1.3., while the SOA/SOC ratio is usually between 1.8 and 2.2 (Zhang et al., 2018; Docherty et al., 2008);

- $AAE_{FF}$ = 1 or measured for optimal values with methods known to the person skilled in the art, for example with levoglucosan or radiocarbon $^{14}$C regression;

- $AAE_{bb}$ = 2 or measured for optimal values with methods known to the person skilled in the art, for example with

levoglucosan or radiocarbon $^{14}$C regression;

- $AAE_{BrC}$ = *calculated numerically with extrapolation of the light absorption on BC at 880 nm; Assumptions: $AAE_{BC}$ is constant in time and BC is the only light absorber at 880 nm:*

$$b_{abs}(880\ nm, t) = b_{abs}^{BC}(880\ nm, t);\ b_{abs}^{BrC}(880\ nm, t) \ == 0$$

| CA component | Measurement or calculation |
|---|---|
| TC, **Cvoc** | First instrument, namely total carbon analyzer, preferably Magee Scientific instrument TCA08 measures total carbon and Cvoc according to known methods, wherein Cvoc measurement is allowed by the TCA08 construction as described below. |
| BC ($b_{abs}(\lambda)$ - absorption coefficient at $7\lambda$) | Second instrument, namely any suitable absorption photometer, preferably Magee Scientific Aethalometer AE33 measures absorption at preferably 7 different wavelengths and thus determines BC according to known methods. The amount of black carbon BC is determined as follows: $$b_{abs}(\lambda, t) = \frac{b_{ATN}(\lambda, t)}{C}$$ $$BC(t) = \frac{b_{abs}^{BC}(880\ nm, t)}{MAC_{BC}(880nm)}$$ |
| BCff, $BC_{bb}$ | From the total BC, BC caused by traffic, i.e., fossil fuel ($BC_{ff}$) and by biomass burning ($BC_{bb}$) can be distinguished using the following calculation (Sandradewi et al., 2008): $$b_{abs}(\lambda, t) = b_{abs}^{ff}(\lambda, t) + b_{abs}^{bb}(\lambda, t),$$ $$b_{abs}^{ff}(\lambda, t) = b_{abs}^{ff}(\lambda_0, t) \cdot \left(\frac{\lambda}{\lambda_0}\right)^{-AAE_{ff}},$$ $$b_{abs}^{bb}(\lambda, t) = b_{abs}^{bb}(\lambda_0, t) \cdot \left(\frac{\lambda}{\lambda_0}\right)^{-AAE_{bb}},$$ $$b_{abs}^{ff}(\lambda, t) = \frac{b_{abs}(\lambda_0, t) - b_{abs}(\lambda, t) \cdot \left(\frac{\lambda_0}{\lambda}\right)^{-AAE_{ff}}}{\left(\frac{\lambda_0}{\lambda}\right)^{-AAE_{bb}} - \left(\frac{\lambda_0}{\lambda}\right)^{-AAE_{ff}}},$$ $$b_{abs}^{bb}(\lambda, t) = \frac{b_{abs}(\lambda_0, t) - b_{abs}(\lambda, t) \cdot \left(\frac{\lambda_0}{\lambda}\right)^{-AAE_{bb}}}{\left(\frac{\lambda_0}{\lambda}\right)^{-AAE_{ff}} - \left(\frac{\lambda_0}{\lambda}\right)^{-AAE_{bb}}}.$$ $$BC_{ff}(t) = \frac{b_{abs}^{ff}(880\ nm, t)}{MAC_{BC}(880nm)}$$ $$BC_{bb}(t) = \frac{b_{abs}^{bb}(880\ nm, t)}{MAC_{BC}(880nm)}.$$ *and* |
| EC | Elemental carbon is determined from the black carbon using the parameter b, which can be taken from the literature as mentioned above: $$b^*BC$$ |

(continued)

| CA component | Measurement or calculation |
|---|---|
| BrC | The wavelength-dependent optical absorption on carbonaceous aerosols can be apportioned to two components - BC and BrC: $$b_{abs}(\lambda, t) = b_{abs}^{BC}(\lambda, t) + b_{abs}^{BrC}(\lambda, t),$$ $$b_{abs}^{BC}(\lambda, t) = b_{abs}^{BC}(\lambda_0, t) \cdot \left(\frac{\lambda}{\lambda_0}\right)^{-AAE_{BC}},$$ $$b_{abs}^{BrC}(\lambda, t) = b_{abs}^{BrC}(\lambda_0, t) \cdot \left(\frac{\lambda}{\lambda_0}\right)^{-AAE_{BrC}(t)}.$$ $$BrC = \frac{b_{abs}^{BrC}(370nm, t)}{MAC_{BrC}(370nm)}$$ |
| OC | Organic carbon is determined from the total carbon and black carbon as measured by the first and the second instrument: $$OC(t) = TC(t) - BC(t).$$ |
| CA | Complete carbonaceous aerosol can be determined using a novel equation allowed by the device according to the invention: $$CA(t) = BC(t) \cdot \left[1 - \left(\frac{OA}{OC}\right)\right] + TC(t) \cdot \left(\frac{OA}{OC}\right)$$ |
| OA | Organic aerosol can be calculated from the organic carbon using the OA/OC ratio taken from the literature as mentioned above: $$OA(t) = \left[\frac{OA}{OC}\right] OC(t)$$ |
| POC, SOC | Primary and secondary organic carbon content of POA and SOA can be determined from the black carbon measurement by using the following equations: $$POC(t) = \left(\frac{OC}{BC}\right)_{prim} \cdot BC(t)$$ $$SOC(t) = OC(t) - POC(t) = OC(t) - \left(\frac{OC}{BC}\right)_{prim} \cdot BC(t)$$ wherein BC tracer method such as described in article Wu and Yu, 2016 (https://doi.org/10.5194/acp-16-5453-2016) is preferably used for optimal $\left(\frac{OC}{BC}\right)_{prim}$ determination. Mean R squared method: BC(t) and POC(t) are emitted from the same source; therefore, they should be well correlated, while the correlation between the BC(t) and SOC(t) should be low due to different formation paths. The $(OC/BC)_{prim}$ ratio is required for the method. It is determined as the minimum R-squared value between SOC(t)-hypothetical and BC(t), where SOC(t)-hypothetical is calculated for every hypothetically possible $(OC/BC)_{prim}$ ratio. |
| POA, SOA | From POC and SOC the primary and secondary organic aerosol can be calculated, wherein the ratio POA/POC and SOA/SOC are taken from the literature as described above: $$POA(t) = POC(t) \cdot \left(\frac{POA}{POC}\right)$$ |

(continued)

| CA component | Measurement or calculation |
|---|---|
| | $$SOA(t) = SOC(t) \cdot \left(\frac{SOA}{SOC}\right)$$ |
| POA$_{BrC}$ SOA$_{BrC}$ | Primary brown carbon and secondary brown carbon can be determined as follows: $$b_{abs}^{BrC,sec}(\lambda, t) = b_{abs}(\lambda, t) - \left(\frac{b_{abs}(\lambda)}{b_{abs}(\lambda_0)}\right)_{prim} \cdot b_{abs}(\lambda_0, t).$$ $$b_{abs}^{BrC,prim}(\lambda, t) = b_{abs}^{BrC}(\lambda, t) - b_{abs}^{BrC,sec}(\lambda, t).$$ $$POA_{BrC}(t) = \frac{b_{abs}^{BrC,prim}(370\ nm, t)}{MAC_{BrC,prim}(370\ nm)}$$ $$SOA_{BrC}(t) = \frac{b_{abs}^{BrC,sec}(370\ nm, t)}{MAC_{BrC,sec}(3700\ nm)}$$ Again, BC tracer method is preferably used to determine optimal $\left(\frac{b_{abs}(\lambda)}{b_{abs}(\lambda_0)}\right)_{prim}$ . MRS method: $b_{abs}^{BrC,prim}(\lambda, t)$ should well correlate with BC(t) because it is emitted from the same source. Optimal $\left(\frac{b_{abs}(\lambda)}{b_{abs}(\lambda_0)}\right)_{prim}$ $\left(\frac{b_{abs}(\lambda)}{b_{abs}(\lambda_0)}\right)_{prim}$ is found as the value where R-squared value between $b_{abs}^{BrC,sec}(\lambda, t)$ -hypotetical and BC(t) has minimum. |
| OAnon-abs | The non-absorbing fraction of organic aerosol can be calculated from total organic aerosol and brown carbon as follows: $$OA_{non-abs} = OA - BrC$$ |
| POAnon-abs SOAnon-abs | Primary and secondary non-light-absorbing OA can be calculated from total POA and POA$_{BrC}$ and total SOA and SOA$_{BrC}$, respectively: $$POA_{non-abs} = POA - POA_{BrC}$$ $$SOA_{non-abs} = SOA - SOA_{BrC}$$ |

The first instrument, namely the total carbon analyzer, samples and measures total carbon in regular time periods, preferably hourly, wherein the second instrument, namely the aethalometer, preferably samples and measures black carbon every minute. These periods may also be altered according to needs of users and location of the device according to the invention. Time resolution of measurements is limited with the time resolution of the total carbon analyser, which is smaller than the time resolution of the absorption photometer. Usual time resolutions are 20 min, 30 min, 60 min, 2h, 4h, 6h, 12h, in 24 h and can be adjusted with regards to aerosol concentration in air - the lower the concentration, the longer sampling time is needed and vice versa. In urban environments a time resolution of 60 min is sufficient. Input parameters needed for calculation are programmed into the device, either any of the instruments or both, or to the processing means, wherein the instruments and/or the processing means are arranged to perform the above calculations. Calculations may be done in real-time, online or in the period post sampling (postprocessing). All components of carbonaceous aerosol are preferably given in mass concentration units [ng/m$^3$].

The device according to the invention provides results for BC$_{ff}$(t), BC$_{bb}$(t), POA$_{BrC}$ (t), SOA$_{BrC}$ (t), $POA_{non-abs}$ (t), and $SOA_{non-abs}$ (t), preferably hourly, however the time period between result readings may also be from 20 minutes to 24 hours. Thus, users of the device obtain very detailed information about aerosol components during different parts of days and nights (diurnal profile for each CA component). If the sampling and measurement periods are different for both instruments, the results are provided in the longer of the periods, while the more frequent data are averaged to the longer period. For example, if the TCA measures total carbon every hour and the aethalometer determines black carbon every minute, the results are provided by the device hourly and the black carbon data are averaged per hour.

A special configuration of TCA08 instrument allows for Cvoc measurement. The configuration of the sampling piping is such that a quartz-fibre filter is provided, which prevents entry of particles into the instrument, while volatile organic compounds (VOC) can pass through the filter and thus reach the instrument for measurement. The TCA08 device thus has an additional two-part housing, within which the mentioned filter supported on one side by a support mesh and on the other side by a metal ring is provided. Adsorption of organic vapours (VOCs) onto filters during aerosol sampling is known as positive sampling artefact and can be used for measurement of ambient carbon content in VOCs (Cvoc).

The method for complete aerosol determination performed by the device according to the invention comprises the following steps:

a) measurements with the first and the second instrument to obtain measurements TC(t) and BC(t);
b) OC calculation from the data obtained in step a);
c) Calculation of $BC_{ff}(t)$, $BC_{bb}(t)$ from the data obtained in step a) by the second instrument, which is any suitable aethalometer, wherein required parameters: $AAE_{ff}$, $AAE_{bb}$ are taken from the literature or measured separately or defined in advance;

d) BrC calculation for light absorption apportionment to $b_{abs}^{BC}(\lambda, t)$ and $b_{abs}^{BrC}(\lambda, t)$; wherein the required parameter is AAEsc and that it is assumed that $b_{abs}^{BrC}(880\ nm,\ t) = 0$;

e) Calculation of POC(t) and SOC(t) from OC(t) obtained in step b);

f) Performing BC tracer method for $b_{abs}^{BrC,prim}(\lambda, t)$ and $b_{abs}^{BrC,sec}(\lambda, t)$

g) Calculation of $POA_{BrC}(t)$ and $SOA_{BrC}(t)$ from $b_{abs}^{BrC,prim}(\lambda, t)$ and $b_{abs}^{BrC,sec}(\lambda, t)$ obtained in step f), wherein the required parameters are $MAC_{BrC,prim}(370\ nm)$ and $MAC_{BrC,sec}(370\ nm)$;

h) Calculation of POA(t) and SOA(t) from POC(t) and SOC(t) obtained in step e), wherein required parameters are (POA/POC) and (SOA/SOC) ratios;

i) Calculation of $POA_{non-abs}(t)$ as POA(t)- $POA_{BrC}(t)$ and calculation of $SOA_{non-abs}(t)$ as SOA(t)- $SOA_{BrC}(t)$; and

j) calculation of total carbonaceous aerosols CA(t) = $BC_{ff}(t)$ + $BC_{bb}(t)$ + $POA_{BrC}(t)$ + $SOA_{BrC}(t)$+ $POA_{non-abs}(t)$ + $SOA_{non-abs}(t)$.

The steps c), d), and e) may be performed in any sequence, wherein other steps are performed as described. Calculation and processing of data is performed by the device (system) or by any of the two instruments, preferably by the total carbon analyzer. The processing protocol may be performed online by an instrument, computer or by postprocessing of measurement data.

The device and the method according to the invention take the advantage of decoupling thermal and optical method into two separate instruments, both dedicated for different measurements. With this, the new method has higher time resolution, no dead time, online loading compensation for BC measurements and is more convenient for field measurements.

Use of the device and the method is important in determination of particles and especially pollutants in air. It has been pointed out on several occasions that beside the particle mass concentration, the chemical composition and size distribution of PM are equally important. Most importantly, BC can affect health and climate, BrC can cause various health effects, wherein PAHs are cancerogeous, and $OA_{non-abs}$ are the largest contribution to the total CA mass and consequently PM. The invention allows simplified determination and complete analysis of carbonaceous aerosols, online and high-time resolution and use of only two instruments.

The device according to the invention may be designed in alternative ways obvious to the skilled person based on the above description, within the scope of the claims. The exemplary embodiments of the invention do not limit the scope of the invention as described herein and defined in the claims.

The invention will be further disclosed based on exemplary embodiments and figures, which show:

Figure 1    Overview of the CA components
Figure 2    Measured and determined CA components by the system according to a possible embodiment
Figure 3    TCA configuration for Cvoc measurement
Figure 4    Visual presentation of the calculation method

Figure 5     Exemplary results obtained with the device according to the invention

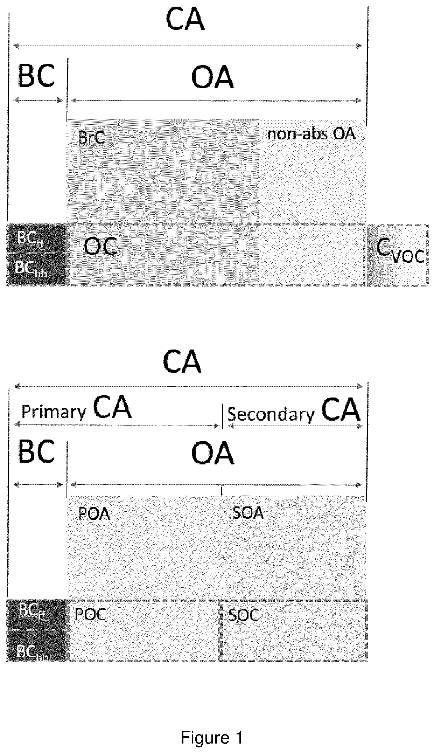

Figure 1 shows an overview of the carbonaceous aerosol components, wherein CA is divided into black carbon (BC) and organic aerosols (OA). The latter can be further divided into brown carbon (BrC) representing organic carbon (OC) and non-absorbing organic aerosols, wherein the source of BC may be traffic (fossil fuel) or biomass burning. Volatile organic compounds may also be present in the CA. With regards to the source, CA may have primary origin or secondary origin, wherein BC is always primary and OA may be primary or secondary as described in the background of the invention.

The device CASS (Carbonaceous Aerosol Speciation System) according to a possible embodiment comprises a first instrument, which is a Total Carbon Analyzer TCA08 and a second instrument, which is an Aethalometer AE33, both by Magee Scientific.

The first instrument collects a sample of atmospheric aerosols on a quartz fibre filter positioned inside a small stainless-steel chamber, at a controlled sampling flow rate, preferably of 16.7 LPM. The default sampling time of TCA08 is 60 min, but can be set from 20 min - 24 h, depending on the ambient aerosol concentrations. TCA08 instrument has two identical parallel channels, through which air-flows are being controlled by ball valves and solenoids, wherein one channel is collecting a sample and the other channel performs analysis of an already collected sample, which is flash-heated to convert all Carbon to $CO_2$. The $CO_2$ concentration is then integrated to give the Total Carbon (TC) content of the sample.

In parallel, the second instrument Magee Scientific Aethalometer® model AE33 is performing an optical attenuation analysis at 7 wavelengths from near UV (370 nm) to near IR range (950 nm) in order to characterize black carbon (BC) aerosols accumulated on a glass-fibre/PTFE filter tape.

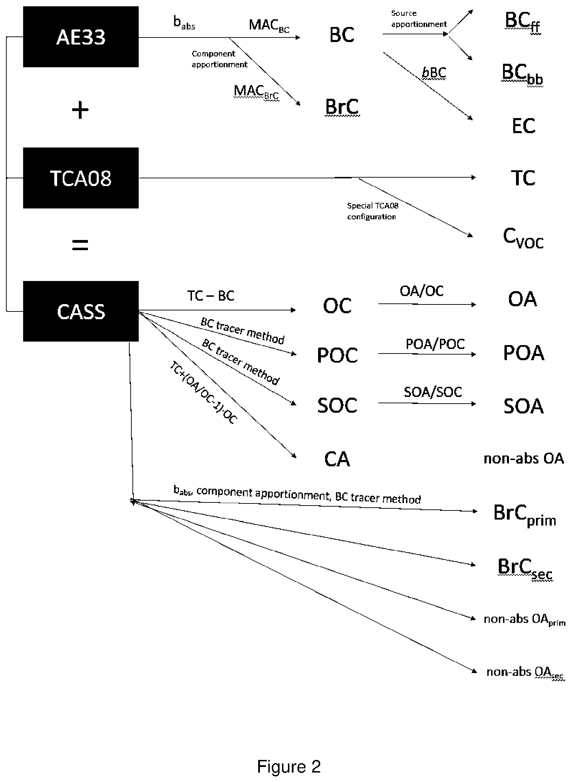

Figure 2 shows which CA components are determined with each of the instruments, wherein AE33 provides BC and BrC, as well as $BC_{ff}$, *$BC_{bb}$* and EC data, while the TCA08 provides TC and Cvoc. From these parameters OC, OA, POC, POA, SOC, SOA, $OA_{non-abs}$, $POA_{non-abs}$, *$SOA_{non-abs}$* as well as total CA can be calculated according to the equations presented above. Processing of acquired data and calculation of remaining CA components is performed hourly by the TCA 08 device, wherein AE33 reports on a defined time-base, which is usually 1 s or 60 s the BC/EC data.

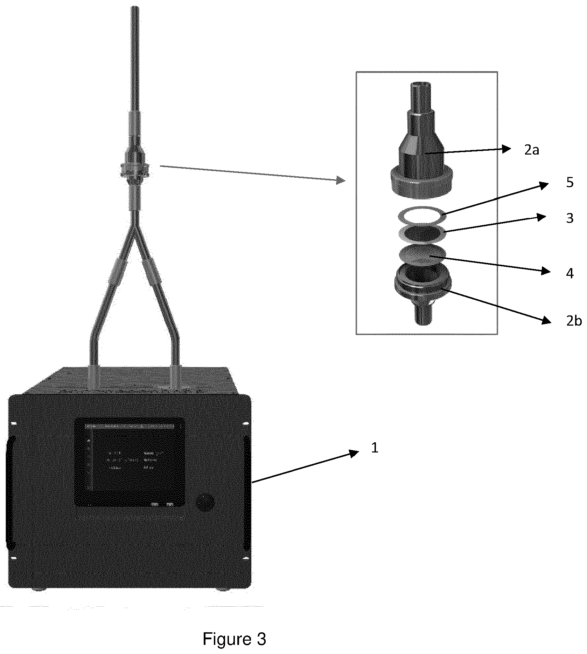

Figure 3 shows the configuration of TCA08 device 1, which allows measurement of Cvoc, wherein a quartz-fibre filter is provided, which prevents entry of particles into the instrument, while volatile organic compounds (VOC) can pass through the filter and thus reach the instrument for measurement. The TCA08 device 1 thus has an additional two-part housing 2a, 2b, within which the mentioned filter 3 supported on one side by a support mesh 4 and on the other side by a metal ring 5 is provided.

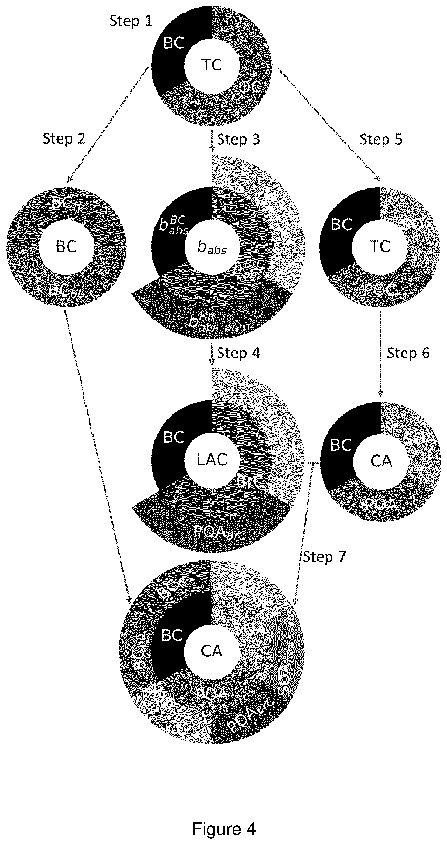

A visual presentation of the calculation method is shown in figure 4 comprises the following steps:

a) measurements with the first and the second instrument to obtain measurements TC(t) and BC(t);

b) OC calculation from the data obtained in step a);

c) Calculation of $BC_{ff}$(t), $BC_{bb}$(t) from the data obtained in step a) by the second instrument, which is any suitable absorption photometer, wherein required parameters: $AAE_{ff}$, $AAE_{bb}$ are taken from the literature or measured separately or defined in advance;

d) BrC calculation for light absorption apportionment to $b_{abs}^{BC}(\lambda, t)$ and $b_{abs}^{BrC}(\lambda, t)$; wherein the required parameter is AAEsc and that it is assumed that $b_{abs}^{BrC}(880\ nm,\ t) = 0$;

e) Calculation of POC(t) and SOC(t) from OC(t) obtained in step b);

f) Performing BC tracer method for $b_{abs}^{BrC,prim}(\lambda, t)$ and $b_{abs}^{BrC,sec}(\lambda, t)$;

g) Calculation of $POA_{BrC}$ (t) and $SOA_{BrC}$ (t) from $b_{abs}^{BrC,prim}(\lambda, t)$ and $b_{abs}^{BrC,sec}(\lambda, t)$ obtained in step f), wherein the required parameters are $MAC_{BrC,prim}$(370 nm) and $MAC_{BrC,sec}$(370 nm);

h) Calculation of POA(t) and SOA(t) from POC(t) and SOC(t) obtained in step e), wherein required parameters are (POA/POC) and (SOA/SOC) ratios;

i) Calculation of *$POA_{non-abs}$* (t) as POA(t)- $POA_{BrC}$ (t) and calculation of *$SOA_{non-abs}$* (t) as SOA(t)- $SOA_{BrC}$(t); and

j) calculation of total carbonaceous aerosols CA(t) = $BC_{ff}$(t) + $BC_{bb}$(t) + $POA_{BrC}$ (t) + $SOA_{BrC}$ (t)+ *$POA_{non-abs}$* (t) + *$SOA_{non-abs}$* (t).

The steps c), d), and e) may be performed in any sequence, wherein other steps are performed as described. In general, until the steps intersect, they may be performed in any order, while each vertical line has to be performed in the sequence shown in figure 4.

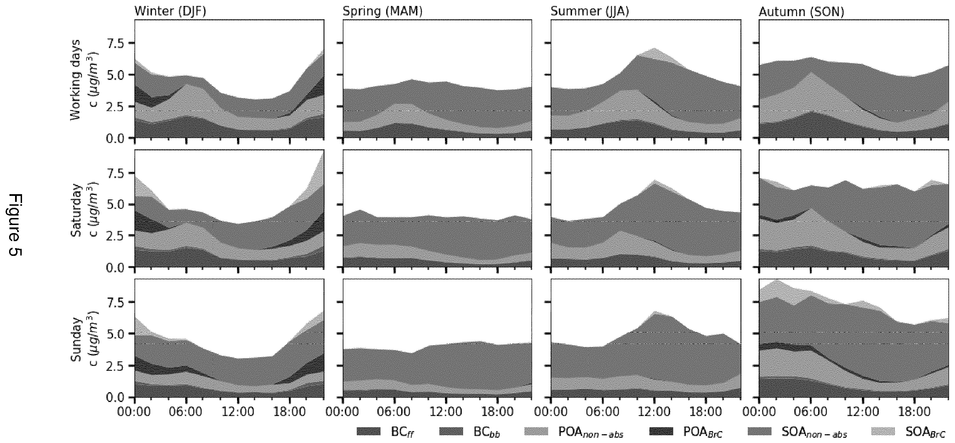

Figure 5 shows exemplary results obtained with the device according to the possible embodiment, wherein it is visible how individual components of the CA vary between seasons and also during the day.

**Claims**

1. A device for complete carbonaceous aerosol analysis in real time, **characterized in that**:

   - the device is a system combining two different instruments, wherein the first instrument measures total carbon (TC) using flash heating of collected aerosol samples and generation of $CO_2$, while the second instrument performs an optical attenuation analysis at multiple wavelengths, preferably 7 wavelengths, in the range from near UV to near IR range, said first instrument being suitable to characterize optical absorption of aerosols accumulated on a glass-fiber/PTFE filter tape,
   - the device, i.e. the system of said instruments, is arranged to collect and process collected data of both instruments, wherein said processing may be performed by any of the instruments or by a separate processing means, computer or computer application, and
   - total carbonaceous aerosols CA(t) are the sum of black carbon originating from fossil fuel ($BC_{ff}$) and by biomass burning ($BC_{bb}$), primary and secondary brown carbon as organic aerosol $POA_{BrC}$ (t) and $SOA_{BrC}$ (t), non absorbing primary organic aerosols ($POA_{non-abs}$ (t)) and non-absorbing secondary aerosols ($SOA_{non-abs}$ (t)), wherein the following calculations are programmed into the device, any of the instruments or both instruments, or to a suitable processing means connected to the device:

   a) the first and the second instrument are configured to measure TC(t) and BC(t), respectively;

   b) organic carbon (OC) is calculated as OC(t) = TC(t) - BC(t);

   c) $BC_{ff}$(t), $BC_{bb}$(t) are calculated from the data obtained in step a) by the second instrument, wherein required parameters: absorption Ångström exponents $AAE_{ff}$, $AAE_{bb}$ are taken from the literature or measured separately or defined in advance;

   d) brown carbon BrC calculation for light absorption apportionment to $b_{abs}^{BC}$ $(\lambda, t)$ and $b_{abs}^{BrC}$ $(\lambda, t)$; wherein the required parameter is $AAE_{BC}$ and it is assumed that $b_{abs}^{BrC}$ $(880\ nm,\ t) = 0$;

   e) performing BC tracer method for $b_{abs}^{BrC,prim}$ $(\lambda, t)$ and $b_{abs}^{BrC,sec}$ $(\lambda, t)$; wherein calculation of primary and secondary organic carbon POC(t) and SOC(t) from OC(t) obtained in step b) is achieved by using the following equations:

$$POC(t) = \left(\frac{OC}{BC}\right)_{prim} \cdot BC(t)$$

$$SOC(t) = OC(t) - POC(t) = OC(t) - \left(\frac{OC}{BC}\right)_{prim} \cdot BC(t) \ ;$$

   f) calculation of $POA_{BrC}$ (t) and $SOA_{BrC}$ (t) from $b_{abs}^{BrC,prim}$ $(\lambda, t)$ and $b_{abs}^{BrC,sec}$ $(\lambda, t)$ obtained in step e), wherein the required parameters are mass absorption cross-section $MAC_{BrC,prim}$(370 nm) and $MAC_{BrC,sec}$(370 nm):

   g) calculation of POA(t) and SOA(t) from POC(t) and SOC(t) obtained in step e), wherein required parameters are (POA/POC) and (SOA/SOC) ratios;

   h) calculation of $POA_{non-abs}$ (t) as POA(t) - $POA_{BrC}$ (t) and calculation of $SOA_{non-abs}$ (t) as SOA(t) - $SOA_{BrC}$ (t); and

   i) calculation of total carbonaceous aerosols CA(t) = $BC_{ff}$(t) + $BC_{bb}$(t) + $POA_{BrC}$ (t) + $SOA_{BrC}$ (t) + $POA_{non-abs}$ (t) + $SOA_{non-abs}$ (t),

   wherein steps c), d) and e) may be performed in any sequence.

2. The device according to claim 1, **characterized in that** the amount of black carbon BC is determined as follows:

$$b_{abs}(t) = \frac{b_{ATN}(t)}{C}$$

$$BC(t) = \frac{b_{abs}^{BC}(880\ nm, t)}{MAC_{BC}(880nm)}$$

wherein ATN is attenuation of light in the filter tape loaded with the sample is calculated as ATN = -100 · ln (I/Io), where I is the detector intensity signal for the measurement spot and I0 the detector signal for the reference spot, C is filtermatrix multi-scattering parameter or enhancement parameter, and MAC is mass absorption cross-section, and BC caused by traffic, i.e., fossil fuel ($BC_{ff}$) and by biomass burning ($BC_{bb}$) can be distinguished using the following calculation, wherein AAE is absorption Ångström exponent:

$$b_{abs}(\lambda, t) = b_{abs}^{ff}(\lambda, t) + b_{abs}^{bb}(\lambda, t),$$

$$b_{abs}^{ff}(\lambda, t) = b_{abs}^{ff}(\lambda_0, t) \cdot \left(\frac{\lambda}{\lambda_0}\right)^{-AAE_{ff}},$$

$$b_{abs}^{bb}(\lambda, t) = b_{abs}^{bb}(\lambda_0, t) \cdot \left(\frac{\lambda}{\lambda_0}\right)^{-AAE_{bb}},$$

$$b_{abs}^{ff}(\lambda) = \frac{b_{abs}(\lambda_0) - b_{abs}(\lambda) \cdot \left(\frac{\lambda_0}{\lambda}\right)^{-AAE_{ff}}}{\left(\frac{\lambda_0}{\lambda}\right)^{-AAE_{bb}} - \left(\frac{\lambda_0}{\lambda}\right)^{-AAE_{ff}}},$$

$$b_{abs}^{bb}(\lambda) = \frac{b_{abs}(\lambda_0) - b_{abs}(\lambda) \cdot \left(\frac{\lambda_0}{\lambda}\right)^{-AAE_{bb}}}{\left(\frac{\lambda_0}{\lambda}\right)^{-AAE_{ff}} - \left(\frac{\lambda_0}{\lambda}\right)^{-AAE_{bb}}}.$$

$$BC_{ff}(t) = \frac{b_{abs}^{ff}(880\ nm, t)}{MAC_{BC}(880nm)} \quad and \quad BC_{bb}(t) = \frac{b_{abs}^{bb}(880\ nm, t)}{MAC_{BC}(880nm)}.$$

3. The device according to claim 1 or claim 2, **characterized in that** the wavelength-dependent optical absorption on carbonaceous aerosols can be apportioned to two components - BC and BrC:

$$b_{abs}(\lambda, t) = b_{abs}^{BC}(\lambda, t) + b_{abs}^{BrC}(\lambda, t),$$

$$b_{abs}^{BC}(\lambda, t) = b_{abs}^{BC}(\lambda_0, t) \cdot \left(\frac{\lambda}{\lambda_0}\right)^{-AAE_{BC}},$$

$$b_{abs}^{BrC}(\lambda, t) = b_{abs}^{BrC}(\lambda_0, t) \cdot \left(\frac{\lambda}{\lambda_0}\right)^{-AAE_{BrC}(t)}.$$

$$BrC = \frac{b_{abs,BrC}}{MAC_{BrC}}$$

4. The device according to any of the preceding claims, **characterized in that** carbonaceous aerosol can be calculated from the organic carbon using the OA/OC ratio taken from the literature using the following equation

$$CA(t) = BC(t) \cdot \left[1 - \left(\frac{OA}{OC}\right)\right] + TC(t) \cdot \left(\frac{OA}{OC}\right)$$

and organic aerosol is determined using a following equation:

$$OA(t) = \left[\frac{OA}{OC}\right] OC(t)$$

5. The device according to any of the preceding claims, **characterized in that** from POC and SOC the primary and secondary organic aerosol can be calculated, wherein the ratio POA/POC and SOA/SOC are taken from the literature

$$POA(t) = POC(t) \cdot \left(\frac{POA}{POC}\right)$$

$$SOA(t) = SOC(t) \cdot \left(\frac{SOA}{SOC}\right)$$

and wherein primary brown carbon and secondary brown carbon can be determined as follows:

$$b_{abs}^{BrC,sec}(\lambda, t) = b_{abs}(\lambda, t) - \left(\frac{b_{abs}(\lambda)}{b_{abs}(\lambda_0)}\right)_{prim} \cdot b_{abs}(\lambda_0, t).$$

$$b_{abs}^{BrC,prim}(\lambda, t) = b_{abs}^{BrC}(\lambda, t) - b_{abs}^{BrC,sec}(\lambda, t).$$

$$POA_{BrC}(t) = \frac{b_{abs}^{BrC,prim}(370\ nm, t)}{MAC_{BrC,prim}(370\ nm)} \quad , \quad SOA_{BrC}(t) = \frac{b_{abs}^{BrC,sec}(370\ nm, t)}{MAC_{BrC,sec}(3700\ nm)}$$

6. The device according to any of the preceding claims, **characterized in that** the total carbon analyzer has a sampling piping provided with a quartz-fibre filter, which prevents entry of particles into the instrument, while volatile organic compounds (VOC) can pass through the filter and thus reach the instrument for measurement.

7. The device according to any of the preceding claims, **characterized in that** the first instrument, namely the total carbon analyzer, is arranged to sample and measure total carbon hourly, wherein the second instrument, namely the aethalometer, is arranged to sample and measure black carbon every minute.

8. The device according to any of the preceding claims, **characterized in that** the device is arranged to perform the calculations in real-time, online or in the period post sampling (postprocessing).

9. The device according to any of the preceding claims, wherein the device is arranged to provide results for $BC_{ff}(t) + BC_{bb}(t) + POA_{BrC}(t) + SOAsrc(t) + POA_{non-abs}(t) + SOA_{non-abs}(t)$ with a time period between result readings from 20 minutes to 24 hours, preferably hourly.

10. A method for complete carbonaceous aerosol analysis in real time performed by the device according to any of the preceding claims, **characterized in that** the method comprises the following steps:

a) measurements with the first and the second instrument to obtain measurements of TC(t) and BC(t), respectively;

b) OC calculation from the data obtained in step a);

c) Calculation of $BC_{ff}(t)$, $BC_{bb}(t)$ from the data obtained in step a) by the second instrument, which is any suitable aethalometer, wherein required parameters: $AAE_{ff}$, $AAE_{bb}$ are taken from the literature or measured separately or defined in advance;

d) BrC calculation for light absorption apportionment to $b_{abs}^{BC}(\lambda, t)$ and $b_{abs}^{BrC}(\lambda, t)$; wherein the required parameter is AAEsc and it is assumed that $b_{abs}^{BrC}(880\ nm,\ t) = 0$;

e) Calculation of POC(t) and SOC(t) from OC(t) obtained in step b) wherein calculation of POC(t) and SOC(t) from OC(t) obtained in step b) is achieved by using the following equations:

$$POC(t) = \left(\frac{OC}{BC}\right)_{prim} \cdot BC(t)$$

$$SOC(t) = OC(t) - POC(t) = OC(t) - \left(\frac{OC}{BC}\right)_{prim} \cdot BC(t)\ ;$$

f) Performing BC tracer method for $b_{abs}^{BrC,prim}(\lambda, t)$ and $b_{abs}^{BrC,sec}(\lambda, t)$;

g) Calculation of $POA_{BrC}(t)$ and $SOA_{BrC}(t)$ from $b_{abs}^{BrC,prim}(\lambda, t)$ and $b_{abs}^{BrC,sec}(\lambda, t)$ obtained in step f), wherein the required parameters are $MAC_{BrC,prim}(370\ nm)$ and $MAC_{BrC,sec}(370\ nm)$;

h) Calculation of POA(t) and SOA(t) from POC(t) and SOC(t) obtained in step e), wherein required parameters are (POA/POC) and (SOA/SOC) ratios;

i) Calculation of $POA_{non-abs}(t)$ as POA(t)- $POA_{BrC}(t)$ and calculation of $SOA_{non-abs}(t)$ as SOA(t)- $SOA_{BrC}(t)$; and

j) calculation of total carbonaceous aerosols $CA(t) = BC_{ff}(t) + BC_{bb}(t) + POA_{BrC}(t) + SOA_{BrC}(t) + POA_{non-abs}(t) + SOA_{non-abs}(t)$,

wherein steps c), d) and e) may be performed in any sequence.

11. The method according to claim 10, wherein calculation and processing of data is performed by the device, i.e., the system, or by any of the two instruments, preferably by the total carbon analyzer, or by separate processing means such as a computer or computer program.

12. The method according to any claim from 10 to 11, wherein calculation and processing of data is performed online by an instrument, computer or by postprocessing of measurement data.

**Patentansprüche**

1. Vorrichtung und Verfahren zur vollständigen Analyse von kohlenstoffhaltigen Aerosolen in Echtzeit, **dadurch gekennzeichnet dass**:

- die Vorrichtung ein System ist, das zwei unterschiedliche Instrumente kombiniert, wobei das erste Instrument den gesamten Kohlenstoff (TC) unter Verwendung von Blitzerhitzung gesammelter Aerosolproben und Erzeugung von $CO_2$ misst, während das zweite Instrument eine optische Dämpfungsanalyse bei mehreren Wellenlängen, vorzugsweise 7 Wellenlängen, im Bereich vom nahen UV- bis zum nahen IR-Bereich durchführt, wobei das erste Instrument geeignet ist, die optische Absorption von Aerosolen zu charakterisieren, die sich auf einem Glasfaser-/PTFE-Filterband angesammelt haben,

- die Vorrichtung, d. h. das System der Instrumente, angeordnet ist, um die erfassten Daten beider Instrumente zu sammeln und zu verarbeiten, wobei die Verarbeitung durch jedes der Instrumente oder durch ein separates Verarbeitungsmittel, einen Computer oder eine Computeranwendung durchgeführt werden kann, und

- die gesamten kohlenstoffhaltigen Aerosole CA(t) die Summe aus Ruß aus fossilen Brennstoffen ($BC_{ff}$) und Biomasseverbrennung ($BC_{bb}$), primärem und sekundärem braunem Kohlenstoff als organisches Aerosol $POA_{BrC}$ (t) und $SOA_{BrC}$ (t), nicht absorbierenden primären organischen Aerosolen ($POA_{non\text{-}abs}$ (t)) und nicht absorbierenden sekundären Aerosolen ($SOA_{non\text{-}abs}$ (t)) ist, wobei die folgenden Berechnungen in die Vorrichtung, in eines der Instrumente oder in beide Instrumente oder in ein geeignetes, mit der Vorrichtung verbundenes Verarbeitungsmittel programmiert werden:

a) das erste und das zweite Instrument sind konfiguriert, um TC(t) bzw. BC(t) zu messen;

b) organischer Kohlenstoff (OC) wird berechnet als OC(t) = TC(t) - BC(t);

c) $BC_{ff}$(t), $BC_{bb}$(t) werden aus den in Schritt a) durch das zweite Instrument erhaltenen Daten berechnet, wobei die erforderlichen Parameter: Absorptions- *Angström Exponenten* $AAE_{ff}$, $AAE_{bb}$ aus der Literatur entnommen oder separat gemessen oder im Voraus definiert werden;

d) Berechnung des braunen Kohlenstoffs BrC zur Aufteilung der Lichtabsorption auf $b_{abs}^{BC}(\lambda, t)$ und $b_{abs}^{BrC}(\lambda, t)$; wobei der erforderliche Parameter $AAE_{BC}$ ist und angenommen wird, dass $b_{abs}^{BrC}(880\ nm,\ t) = 0$;

e) Durchführen des BC-Tracerverfahrens für $b_{abs}^{BrC,prim}(\lambda, t)$ und $b_{abs}^{BrC,sec}(\lambda, t)$; wobei die Berechnung des primären und sekundären organischen Kohlenstoffs POC(t) und SOC(t) aus dem in Schritt b) erhaltenen OC(t) unter Verwendung der folgenden Gleichungen erfolgt:

$$POC(t) = \left(\frac{OC}{BC}\right)_{prim} \cdot BC(t)$$

$$SOC(t) = OC(t) - POC(t) = OC(t) - \left(\frac{OC}{BC}\right)_{prim} \cdot BC(t) ;$$

f) Berechnung von $POA_{BrC}$ (t) und $SOA_{BrC}$ (t) aus $b_{abs}^{BrC,prim}(\lambda, t)$ und $b_{abs}^{BrC,sec}(\lambda, t)$, die in Schritt e) erhalten wurden, wobei die erforderlichen Parameter der Massenabsorptionsquerschnitt $MAC_{BrC,prim}$(370 nm) und $MAC_{BrC,sec}$(370 nm)sind;

g) Berechnung von POA(t) und SOA(t) aus POC(t) und SOC(t), die in Schritt e) erhalten wurden, wobei die erforderlichen Parameter die Verhältnisse (POA/POC) und (SOA/SOC) sind;

h) Berechnung von *POA_{non-abs}* (t) als POA(t)- $POA_{BrC}$ (t), und Berechnung von *SOA_{non-abs}* (t) als SOA(t)- $SOA_{BrC}$ (t); und

i) Berechnung der gesamten kohlenstoffhaltigen Aerosole CA(t) = $BC_{ff}$(t) + $BC_{bb}$(t) + $POA_{BrC}$ (t) + $SOA_{BrC}$ (t) + *POA_{non-abs}* (t) + *SOA_{non-abs}* (t),

wobei Schritte c), d) und e) in beliebiger Reihenfolge durchgeführt werden können.

**2.** Vorrichtung nach Anspruch 1, **dadurch gekennzeichnet, dass** die Menge an Ruß BC wie folgt bestimmt wird:

$$b_{abs}(t) = \frac{b_{ATN}(t)}{C}$$

$$BC(t) = \frac{b_{abs}^{BC}(880\ nm, t)}{MAC_{BC}(880\ nm)}$$

wobei ATN die Lichtdämpfung im mit der Probe beladenen Filterband ist und berechnet wird als ATN = -100 · ln $(I/I_0)$, wobei I das Detektorintensitätssignal für den Messpunkt und I0 das Detektorsignal für den Referenzpunkt ist, C der Filtermatrix-Mehrfachstreuungsparameter oder Verstärkungsparameter ist, und MAC der Massenabsorptionsquerschnitt ist,
und BC, die durch Verkehr, d. h. fossile Brennstoffe ($BC_{ff}$), und durch Verbrennung von Biomasse ($BC_{bb}$) verursacht wird, kann unter Verwendung der folgenden Berechnung unterschieden werden, wobei AAE der Absorptions-Angström-Exponent ist:

$$b_{abs}(\lambda, t) = b_{abs}^{ff}(\lambda, t) + b_{abs}^{bb}(\lambda, t),$$

$$b_{abs}^{ff}(\lambda, t) = b_{abs}^{ff}(\lambda_0, t) \cdot \left(\frac{\lambda}{\lambda_0}\right)^{-AAE_{ff}},$$

$$b_{abs}^{bb}(\lambda, t) = b_{abs}^{bb}(\lambda_0, t) \cdot \left(\frac{\lambda}{\lambda_0}\right)^{-AAE_{bb}},$$

$$b_{abs}^{ff}(\lambda) = \frac{b_{abs}(\lambda_0) - b_{abs}(\lambda) \cdot \left(\frac{\lambda_0}{\lambda}\right)^{-AAE_{ff}}}{\left(\frac{\lambda_0}{\lambda}\right)^{-AAE_{bb}} - \left(\frac{\lambda_0}{\lambda}\right)^{-AAE_{ff}}},$$

$$b_{abs}^{bb}(\lambda) = \frac{b_{abs}(\lambda_0) - b_{abs}(\lambda) \cdot \left(\frac{\lambda_0}{\lambda}\right)^{-AAE_{bb}}}{\left(\frac{\lambda_0}{\lambda}\right)^{-AAE_{ff}} - \left(\frac{\lambda_0}{\lambda}\right)^{-AAE_{bb}}}.$$

$$BC_{ff}(t) = \frac{b_{abs}^{ff}(880\,nm, t)}{MAC_{BC}(880\,nm)} \quad und \quad BC_{bb}(t) = \frac{b_{abs}^{bb}(880\,nm, t)}{MAC_{BC}(880\,nm)}.$$

3. Die Vorrichtung nach Anspruch 1 oder Anspruch 2, **dadurch gekennzeichnet, dass** die wellenlängenabhängige optische Absorption an kohlenstoffhaltigen Aerosolen auf zwei Komponenten - BC und BrC - aufgeteilt werden kann:

$$b_{abs}(\lambda, t) = b_{abs}^{BC}(\lambda, t) + b_{abs}^{BrC}(\lambda, t),$$

$$b_{abs}^{BC}(\lambda, t) = b_{abs}^{BC}(\lambda_0, t) \cdot \left(\frac{\lambda}{\lambda_0}\right)^{-AAE_{BC}},$$

$$b_{abs}^{BrC}(\lambda, t) = b_{abs}^{BrC}(\lambda_0, t) \cdot \left(\frac{\lambda}{\lambda_0}\right)^{-AAE_{BrC}(t)}.$$

$$BrC = \frac{b_{abs,\,BrC}}{MAC_{BrC}}$$

4. Die Vorrichtung nach einem der vorstehenden Ansprüche, **dadurch gekennzeichnet, dass** kohlenstoffhaltiges Aerosol aus dem organischen Kohlenstoff unter Verwendung des aus der Literatur entnommenen OA/OC-Verhältnisses unter Verwendung der folgenden Gleichung berechnet werden kann

$$CA(t) = BC(t) \cdot \left[1 - \left(\frac{OA}{OC}\right)\right] + TC(t) \cdot \left(\frac{OA}{OC}\right)$$

und organisches Aerosol unter Verwendung einer folgenden Gleichung bestimmt werden kann:

$$OA(t) = \left[\frac{OA}{OC}\right] OC(t)$$

**5.** Die Vorrichtung nach einem der vorstehenden Ansprüche, **dadurch gekennzeichnet, dass** aus POC und SOC das primäre und sekundäre organische Aerosol berechnet werden kann, wobei das Verhältnis POA/POC und SOA/SOC aus der Literatur entnommen werden kann

$$POA(t) = POC(t) \cdot \left(\frac{POA}{POC}\right)$$

$$SOA(t) = SOC(t) \cdot \left(\frac{SOA}{SOC}\right)$$

und wobei der primäre braune Kohlestoff und der sekundäre braune Kohlenstoff wie folgt bestimmt werden können:

$$b_{abs}^{BrC,sec}(\lambda, t) = b_{abs}(\lambda, t) - \left(\frac{b_{abs}(\lambda)}{b_{abs}(\lambda_0)}\right)_{prim} \cdot b_{abs}(\lambda_0, t).$$

$$b_{abs}^{BrC,prim}(\lambda, t) = b_{abs}^{BrC}(\lambda, t) - b_{abs}^{BrC,sec}(\lambda, t).$$

$$POA_{BrC}(t) = \frac{b_{abs}^{BrC,prim}(370\ nm, t)}{MAC_{BrC,prim}(370\ nm)} \quad , \quad SOA_{BrC}(t) = \frac{b_{abs}^{BrC,sec}(370\ nm, t)}{MAC_{BrC,sec}(3700\ nm)}$$

**6.** Die Vorrichtung nach einem der vorstehenden Ansprüche, **dadurch gekennzeichnet, dass** der Gesamtkohlenstoffanalysator eine mit einem Quarzfaserfilter versehene Probenahmeleitung aufweist, die das Eindringen von Partikeln in das Instrument verhindert, während flüchtige organische Verbindungen (VOC) den Filter passieren und somit zur Messung das Instrument erreichen können.

**7.** Die Vorrichtung nach einem der vorstehenden Ansprüche, **dadurch gekennzeichnet, dass** das erste Instrument, d. h. der Gesamtkohlenstoffanalysator, angeordnet ist, um stündlich Proben des gesamten Kohlenstoffs zu entnehmen und zu messen, während das zweite Instrument, d. h. das Aethalometer, angeordnet ist, um jede Minute Proben des Rußes zu entnehmen und zu messen.

**8.** Die Vorrichtung nach einem der vorstehenden Ansprüche, **dadurch gekennzeichnet, dass** die Vorrichtung angeordnet ist, um die Berechnungen in Echtzeit, online oder in der Zeit nach der Probenentnahme (Nachbearbeitung) durchzuführen.

**9.** Die Vorrichtung nach einem der vorstehenden Ansprüche, wobei die Vorrichtung angeordnet ist, um Ergebnisse für $BC_{ff}(t) + BC_{bb}(t) + POA_{BrC}(t) + SOA_{BrC}(t) + POA_{non\text{-}abs}(t) + SOA_{non\text{-}abs}(t)$ bereitzustellen, mit einem Zeitraum zwischen den Ergebnisablesungen von 20 Minuten bis 24 Stunden, vorzugsweise stündlich.

**10.** Verfahren zur vollständigen Analyse kohlenstoffhaltiger Aerosole in Echtzeit, das durch die Vorrichtung nach einem der vorstehenden Ansprüche durchgeführt wird, **dadurch gekennzeichnet, dass** das Verfahren die folgenden Schritte umfasst:

a) Messungen mit dem ersten und dem zweiten Instrument, um Messungen von TC(t) bzw. BC(t) zu erhalten;
b) OC-Berechnung aus den in Schritt a) erhaltenen Daten;
c) Berechnung von $BC_{ff}(t)$, $BC_{bb}(t)$ aus den in Schritt a) erhaltenen Daten durch das zweite Instrument, bei dem es sich um ein beliebiges geeignetes Aethalometer handelt, wobei die erforderlichen Parameter: $AAE_{ff}$, $AAE_{bb}$ aus der Literatur entnommen oder separat gemessen oder im Voraus definiert werden;

d) BrC-Berechnung zur Aufteilung der Lichtabsorption auf $b_{abs}^{BC}(\lambda, t)$ und $b_{abs}^{BrC}(\lambda, t)$; wobei der erforderliche Parameter $AAE_{BC}$ ist und angenommen wird, dass $b_{abs}^{BrC}(880\ nm,\ t) = 0$;

e) Berechnung von POC(t) und SOC(t) aus dem in Schritt b) erhaltenen OC(t), wobei die Berechnung von POC(t) und SOC(t) aus dem in Schritt b) erhaltenen OC(t) unter Verwendung der folgenden Gleichungen erfolgt:

$$POC(t) = \left(\frac{OC}{BC}\right)_{prim} \cdot BC(t)$$

$$SOC(t) = OC(t) - POC(t) = OC(t) - \left(\frac{OC}{BC}\right)_{prim} \cdot BC(t);$$

f) Durchführen des BC-Tracerverfahrens für $b_{abs}^{BrC,prim}(\lambda, t)$ und $b_{abs}^{BrC,sec}(\lambda, t)$;

g) Berechnung von $POA_{BrC}(t)$ und $SOA_{BrC}(t)$ aus $b_{abs}^{BrC,prim}(\lambda, t)$ und $b_{abs}^{BrC,sec}(\lambda, t)$, die in Schritt f) erhalten wurden, wobei die erforderlichen Parameter $MAC_{BrC,prim}$(370 nm) und $MAC_{BrC,sec}$(370 nm) sind;
h) Berechnung von POA(t) und SOA(t) aus POC(t) und SOC(t), die in Schritt e) erhalten wurden, wobei die erforderlichen Parameter die Verhältnisse (POA/POC) und (SOA/SOC) sind;
i) Berechnung von $POA_{non\text{-}abs}$ (t) als POA(t) - $POA_{BrC}$ (t), und Berechnung von $SOA_{non\text{-}abs}$ (t) als SOA(t) - $SOA_{BrC}$ (t); und
j) Berechnung der gesamten kohlenstoffhaltigen Aerosole CA(t) = $BC_{ff}$(t) + $BC_{bb}$(t) + $POA_{BrC}$ (t) + $SOA_{BrC}$ (t) + $POA_{non\text{-}abs}$ (t) + $SOA_{non\text{-}abs}$ (t),

wobei Schritte c), d) und e) in beliebiger Reihenfolge durchgeführt werden können.

11. Das Verfahren nach Anspruch 10, wobei die Berechnung und Verarbeitung der Daten durch die Vorrichtung, d. h. das System, oder durch eines der beiden Instrumente, vorzugsweise durch den Gesamtkohlenstoffanalysator, oder durch separate Verarbeitungsmittel wie einen Computer oder ein Computerprogramm durchgeführt wird.

12. Das Verfahren nach einem der Ansprüche 10 bis 11, wobei die Berechnung und Verarbeitung der Daten online durch ein Instrument, einen Computer oder durch Nachbearbeitung der Messdaten erfolgt.

**Revendications**

1. Dispositif et procédé d'analyse complète d'aérosol carboné en temps réel, **caractérisés par le fait que** :

- le dispositif est un système combinant deux instruments différents, le premier instrument mesurant le carbone total (TC) en utilisant un chauffage rapide des échantillons d'aérosol recueillis et la génération de $CO_2$, tandis que le second instrument effectue une analyse de l'atténuation optique à plusieurs longueurs d'onde, de préférence 7 longueurs d'onde, allant de l'ultraviolet proche au proche infrarouge, ledit premier instrument étant capable de déterminer l'absorption optique des aérosols accumulés sur une bande filtrante en fibres de verre/PTFE,
- le dispositif, c.-à-d. le système desdits instruments, est conçu pour recueillir et traiter les données collectées des deux instruments, où ledit traitement peut être réalisé par n'importe lequel des instruments ou par un traitement séparé, un ordinateur ou une application informatique, et
- les aérosols carbonés totaux CA(t) sont la somme du carbone suie provenant des combustibles fossiles ($BC_{ff}$) et de la combustion de biomasse ($BC_{bb}$), des carbones bruns primaires et secondaires sous forme d'aérosols organiques $POA_{BrC}$ (t) et $SOA_{BrC}$(t), des aérosols organiques primaires non absorbants ($POA_{non\text{-}abs}$ (t)) et des aérosols secondaires non-absorbants (SOA$_{non-abs}$ (t)), où les calculs suivants sont programmés dans le dispositif, un des deux instruments ou les deux, ou dans un moyen de traitement approprié connecté au dispositif :

a) le premier et le second instrument sont configurés pour mesurer le TC(t) et le BC(t), respectivement ;

b) le carbone organique (OC) est calculé comme suit : OC(t) = TC(t) - BC(t) ;

c) BC$_{ff}$(t) et BC$_{bb}$(t) sont calculés à partir des données obtenues à l'étape a) par le second instrument, où les paramètres requis : les coefficients *d'Angström d'absorption* AAE$_{ff}$ et AAE$_{bb}$ sont pris dans la littérature scientifique ou mesurés séparément ou définis à l'avance ;

d) Le calcul du carbone brun BrC pour la répartition de l'absorption de la lumière en $b_{abs}^{BC} (\lambda, t)$ et $b_{abs}^{BrC} (\lambda, t)$ ; où le paramètre requis est AAE$_{BC}$ et on part du principe que $b_{abs}^{BrC} (880\ nm,\ t) = 0$ ;

e) la réalisation de la méthode de traçage de BC pour $b_{abs}^{BrC,prim} (\lambda, t)$ et $b_{abs}^{BrC,sec} (\lambda, t)$ ; où le calcul des carbones organiques primaire et secondaire POC(t) et SOC(t) à partir de OC(t) obtenu à l'étape b) est effectué en utilisant les équations suivantes :

$$POC(t) = \left(\frac{OC}{BC}\right)_{prim} \cdot BC(t)$$

$$SOC(t) = OC(t) - POC(t) = OC(t) - \left(\frac{OC}{BC}\right)_{prim} \cdot BC(t) ;$$

f) le calcul de POA$_{BrC}$ (t) et de SOA$_{BrC}$ (t) à partir de $b_{abs}^{BrC,prim} (\lambda, t)$ et de $b_{abs}^{BrC,sec} (\lambda, t)$ obtenus à l'étape e), où les paramètres requis sont les sections efficaces d'absorption massique MAC$_{BrC,prim}$(370 nm) et MAC$_{BrC,sec}$(370 nm) ;

g) le calcul de POA(t) et de SOA(t) à partir de POC(t) et SOC(t) obtenus à l'étape e), où les paramètres requis sont les rapports (POA/POC) et (SOA/SOC) ;

h) le calcul de *POA$_{non-abs}$* (t) en tant que POA(t)- POA$_{BrC}$ (t) et le calcul de *SOA$_{non-abs}$* (t) en tant que SOA(t)-SOA$_{BrC}$(t) ; et

i) le calcul des aérosols carbonés totaux CA(t) = BC$_{ff}$(t) + BC$_{bb}$(t) + POA$_{BrC}$ (t) + SOA$_{BrC}$ (t)+ *POA$_{non-abs}$* (t) + *SOA$_{non-abs}$* (t),

où les étapes c), d) et e) peuvent être effectuées dans n'importe quel ordre.

2. Le dispositif selon la revendication 1, **caractérisé par le fait que** la quantité de carbone suie BC est déterminée de la façon suivante :

$$b_{abs}(t) = \frac{b_{ATN}(t)}{C}$$

$$BC(t) = \frac{b_{abs}^{BC}(880\ nm, t)}{MAC_{BC}(880\ nm)}$$

où ATN est l'atténuation de la lumière dans la bande filtrante où l'échantillon est déposé, et est calculée en tant que ATN = -100 · ln (I/I$_0$), où I est le signal d'intensité du détecteur pour la zone de mesure et I0 le signal du détecteur pour la zone de référence, C est le paramètre de multi-dispersion ou paramètre d'intensification de la matrice filtrante, et MAC est la section efficace d'absorption massique,

et le BC causé par la circulation, c.-à-d. le combustible fossile (BC$_{ff}$), et par la combustion de biomasse (BC$_{bb}$) peut être déterminé au moyen du calcul suivant, où AAE est le coefficient d'Angström d'absorption :

$$b_{\text{abs}}(\lambda, t) = b_{\text{abs}}^{\text{ff}}(\lambda, t) + b_{\text{abs}}^{\text{bb}}(\lambda, t),$$

$$b_{\text{abs}}^{\text{ff}}(\lambda, t) = b_{\text{abs}}^{\text{ff}}(\lambda_0, t) \cdot \left(\frac{\lambda}{\lambda_0}\right)^{-AAE_{ff}},$$

$$b_{\text{abs}}^{\text{bb}}(\lambda, t) = b_{\text{abs}}^{\text{bb}}(\lambda_0, t) \cdot \left(\frac{\lambda}{\lambda_0}\right)^{-AAE_{bb}},$$

$$b_{\text{abs}}^{\text{ff}}(\lambda) = \frac{b_{\text{abs}}(\lambda_0) - b_{\text{abs}}(\lambda) \cdot \left(\frac{\lambda_0}{\lambda}\right)^{-AAE_{ff}}}{\left(\frac{\lambda_0}{\lambda}\right)^{-AAE_{bb}} - \left(\frac{\lambda_0}{\lambda}\right)^{-AAE_{ff}}},$$

$$b_{\text{abs}}^{\text{bb}}(\lambda) = \frac{b_{\text{abs}}(\lambda_0) - b_{\text{abs}}(\lambda) \cdot \left(\frac{\lambda_0}{\lambda}\right)^{-AAE_{bb}}}{\left(\frac{\lambda_0}{\lambda}\right)^{-AAE_{ff}} - \left(\frac{\lambda_0}{\lambda}\right)^{-AAE_{bb}}}.$$

$$BC_{\text{ff}}(t) = \frac{b_{\text{abs}}^{\text{ff}}(880\,\text{nm}, t)}{MAC_{BC}(880\,\text{nm})} \quad \text{et} \quad BC_{\text{bb}}(t) = \frac{b_{\text{abs}}^{\text{bb}}(880\,\text{nm}, t)}{MAC_{BC}(880\,\text{nm})}.$$

3. Le dispositif selon la revendication 1 ou la revendication 2, **caractérisé par le fait que** l'absorption optique dépendant de la longueur d'onde sur les aérosols carbonés peut être répartie en deux composants : BC et BrC :

$$b_{\text{abs}}(\lambda, t) = b_{\text{abs}}^{\text{BC}}(\lambda, t) + b_{\text{abs}}^{\text{BrC}}(\lambda, t),$$

$$b_{\text{abs}}^{\text{BC}}(\lambda, t) = b_{\text{abs}}^{\text{BC}}(\lambda_0, t) \cdot \left(\frac{\lambda}{\lambda_0}\right)^{-AAE_{BC}},$$

$$b_{\text{abs}}^{\text{BrC}}(\lambda, t) = b_{\text{abs}}^{\text{BrC}}(\lambda_0, t) \cdot \left(\frac{\lambda}{\lambda_0}\right)^{-AAE_{BrC}(t)}.$$

$$BrC = \frac{b_{\text{abs,BrC}}}{MAC_{BrC}}$$

4. Le dispositif selon l'une quelconque des revendications précédentes, **caractérisé par le fait que** l'aérosol carboné peut être calculé à partir du carbone organique en utilisant le rapport OA/OC trouvé dans la littérature scientifique à l'aide de l'équation suivante

$$CA(t) = BC(t) \cdot \left[1 - \left(\frac{OA}{OC}\right)\right] + TC(t) \cdot \left(\frac{OA}{OC}\right)$$

et l'aérosol organique est déterminé grâce à l'équation suivante :

$$OA(t) = \left[\frac{OA}{OC}\right] OC(t)$$

**5.** Le dispositif selon l'une quelconque des revendications suivantes, **caractérisé par le fait qu'**à partir de POC et de SOC, les aérosols organiques primaire et secondaire peuvent être calculés, où les rapports POA/POC et SOA/SOC sont trouvés dans la littérature scientifique

$$POA(t) = POC(t) \cdot \left(\frac{POA}{POC}\right)$$

$$SOA(t) = SOC(t) \cdot \left(\frac{SOA}{SOC}\right)$$

et où le carbone brun primaire et le carbone brun secondaire peuvent être déterminés de la façon suivante :

$$b_{abs}^{BrC,sec}(\lambda, t) = b_{abs}(\lambda, t) - \left(\frac{b_{abs}(\lambda)}{b_{abs}(\lambda_0)}\right)_{prim} \cdot b_{abs}(\lambda_0, t).$$

$$b_{abs}^{BrC,prim}(\lambda, t) = b_{abs}^{BrC}(\lambda, t) - b_{abs}^{BrC,sec}(\lambda, t).$$

$$POA_{BrC}(t) = \frac{b_{abs}^{BrC,prim}(370\,nm, t)}{MAC_{BrC,prim}(370\,nm)} \quad , \quad SOA_{BrC}(t) = \frac{b_{abs}^{BrC,sec}(370\,nm, t)}{MAC_{BrC,sec}(3700\,nm)}$$

**6.** Le dispositif selon l'une quelconque des revendications précédentes, **caractérisé par le fait que** l'analyseur de carbone total a un prélèvement d'échantillon fourni par un filtre en fibres de quartz, qui empêche l'entrée de particules dans l'instrument, tandis que les composés organiques volatiles (VOC) peuvent passer à travers le filtre et ainsi atteindre l'instrument pour la mesure.

**7.** Le dispositif selon l'une quelconque des revendications précédentes, **caractérisé par le fait que** le premier instrument, à savoir l'analyseur de carbone total, est conçu de façon à échantillonner et mesurer le carbone total toutes les heures, alors que le second instrument, à savoir l'aéthalomètre, est conçu pour échantillonner et mesurer le carbone suie toutes les minutes.

**8.** Le dispositif selon l'une quelconque des revendications précédentes, **caractérisé par le fait que** le dispositif est conçu pour effectuer les calculs en temps réel, en ligne ou à un moment post-échantillonnage (post-traitement).

**9.** Le dispositif selon l'une quelconque des revendications précédentes, où le dispositif est conçu pour fournir des résultats pour $BC_{ff}(t) + BC_{bb}(t) + POA_{BrC}(t) + SOA_{BrC}(t) + POA_{non-abs}(t) + SOA_{non-abs}(t)$, avec une période de temps entre les lectures de résultats allant de 20 minutes à 24 heures, de préférence toutes les heures.

**10.** Une méthode pour l'analyse complète en temps réel des aérosols carbonés, effectuée par le dispositif selon l'une quelconque des revendications précédentes, **caractérisée par le fait que** la méthode comprend les étapes suivantes :

a) des mesures avec le premier et le second instrument pour obtenir des mesures du TC(t) et du BC(t), respectivement ;
b) le calcul de l'OC à partir des données obtenues à l'étape a) ;
c) le calcul de $BC_{ff}(t)$, $BC_{bb}(t)$ à partir des données obtenues à l'étape a) par le second instrument, qui est tout aéthalomètre approprié, où les paramètres requis : $AAE_{ff}$ et $AAE_{bb}$, sont pris dans la littérature scientifique ou mesurés séparément ou définis à l'avance ;

d) Le calcul du BrC pour la répartition de l'absorption de la lumière en $b_{abs}^{BC}(\lambda, t)$ et $b_{abs}^{BrC}(\lambda, t)$ ; où le paramètre requis est $AAE_{BC}$ et on part du principe que $b_{abs}^{BrC}(880\ nm,\ t) = 0$ ;

e) Le calcul de POC(t) et de SOC(t) à partir de OC(t) obtenu à l'étape b), où le calcul de POC(t) et SOC(t) à partir de OC(t) obtenu à l'étape b) est effectué en utilisant les équations suivantes :

$$POC(t) = \left(\frac{OC}{BC}\right)_{prim} \cdot BC(t)$$

$$SOC(t) = OC(t) - POC(t) = OC(t) - \left(\frac{OC}{BC}\right)_{prim} \cdot BC(t) ;$$

f) La réalisation de la méthode de traçage de BC pour $b_{abs}^{BrC,prim}(\lambda, t)$ et $b_{abs}^{BrC,sec}(\lambda, t)$ ;

g) Le calcul de $POA_{BrC}(t)$ et de $SOA_{BrC}(t)$ à partir de $b_{abs}^{BrC,prim}(\lambda, t)$ et de $b_{abs}^{BrC,sec}(\lambda, t)$ obtenus à l'étape f), où les paramètres requis sont $MAC_{BrC,prim}(370\ nm)$ et $MAC_{BrC,sec}(370\ nm)$ ;

h) Le calcul de POA(t) et de SOA(t) à partir de POC(t) et SOC(t) obtenus à l'étape e), où les paramètres requis sont les rapports (POA/POC) et (SOA/SOC) ;

i) le calcul de $POA_{non\text{-}abs}(t)$ en tant que POA(t)- $POA_{BrC}(t)$ et le calcul de $SOA_{non\text{-}abs}(t)$ en tant que SOA(t)- $SOA_{BrC}(t)$ ; et

j) le calcul des aérosols carbonés totaux CA(t) = $BC_{ff}(t)$ + $BC_{bb}(t)$ + $POA_{BrC}(t)$ + $SOA_{BrC}(t)$ + $POA_{non\text{-}abs}(t)$ + $SOA_{non\text{-}abs}(t)$,

où les étapes c), d) et e) peuvent être effectuées dans n'importe quel ordre.

11. La méthode selon la revendication 10, où le calcul et le traitement des données sont réalisés par le dispositif, c.-à-d. le système, ou par n'importe lequel des deux instruments, de préférence par l'analyseur de carbone total, ou par des moyens de traitement séparés, tels qu'un ordinateur ou un programme informatique.

12. La méthode selon la revendication 10 ou la revendication 11, où le calcul et le traitement des données sont effectués en ligne par un instrument, un ordinateur ou par post-traitement des données de mesure.

Figure 1

Figure 2

Figure 3

Figure 4

Figure 5

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019277819 A

- CN 209182229

**Non-patent literature cited in the description**

- **HUNTZICKER, J. J.**; **JOHNSON, R. L**; **SHAH, J. J.**; **CARY, R. A.** *Analysis of Organic and Elemental Carbon in Ambient Aerosols by a thermal-Optical Method*, 1982, 79-88